## Europäisches Patentamt
## European Patent Office
## Office européen des brevets

(11) Publication number: **0 150 066**
A2

(12)

# EUROPEAN PATENT APPLICATION

(21) Application number: **85100549.6**

(22) Date of filing: **19.01.85**

(51) Int. Cl.⁴: **C 07 K 15/26**
**C 07 K 3/12**

(30) Priority: **23.01.84 JP 10858/84**
**09.01.85 JP 2586/85**

(43) Date of publication of application:
**31.07.85 Bulletin 85/31**

(84) Designated Contracting States:
**AT BE CH DE FR GB IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Nara, Kiyoshi**
**53-12, Katsurainuicho**
**Nishikyo-ku Kyoto 615(JP)**

(72) Inventor: **Honda, Susumi**
**2-1, Tenjincho 2-chome**
**Takatsuki Osaka 569(JP)**

(74) Representative: **von Kreisler, Alek, Dipl.-Chem. et al,**
**Deichmannhaus am Hauptbahnhof**
**D-5000 Köln 1(DE)**

(54) Highly solubilized protein and production thereof.

(57) A highly concentrated aqueous solution of human γ-interferon is produced by removing a protein-denaturing agent from a diluted aqueous solution of human γ-interferon containing the protein-denaturing agent, aging the resulting solution in situ and concentrating the same.

Croydon Printing Company Ltd

EP 0 150 066 A2

High Solubilized Protein and Production Thereof

This invention relates to a highly solubilized protein and production thereof.

More concretely, this invention relates to a highly concentrated aqueous solution of human $\gamma$-interferon.

Interferons (hereinafter sometimes abbreviated as IFNs) are proteins produced by higher animal cells upon induction by stimulation by viruses, nucleic acids, etc., and have antiviral and antitumor activities, among others.

For human interferons, three types differing in characteristic properties are currently known, namely $\alpha$, $\beta$ and $\gamma$ types.

Studies on $\alpha$-type interferon (hereinafter abbreviated as IFN-$\alpha$) and $\beta$-type interferon (hereinafter abbreviated as IFN-$\beta$) have advanced to a considerable extent. Thus, methods of purification thereof have been developed and their properties have become known to a good extent.

γ-Type interferon (hereinafter sometimes abbreviated as IFN-γ) is produced by immunocompetent cells under circumstances such that blast transformation of lymphocytes or production of lymphokines can take place, and accordingly it is also called immune interferon. IFN-γ is said to have higher antiproliferative or antitumor activity as compared with IFN-α and IFN-β and therefore more expected of from the clinical application standpoint. However, due to various limitations, such as necessity for fresh lymphocytes for the production thereof, any efficient production systems have not been established yet. In addition, it is suggested that, in different experiment systems, different cell species can possibly produce different molecular species of IFN-γ, and their structures and properties still remain unknown in many aspects.

The present inventors were engaged in research and development works aiming at developing a technology for purifying human IFN-γ produced by utilizing a genetic engineering technique. In the course of study, they found that human IFN-γ has a strong tendency to polymerization, whereby its purification is rendered very difficult. For solving this problem, they have developed a method for producing monomeric human IFN-γ which comprises performing gel filtration under the coexistence of a reducing sulfur compound and a protein-denaturing agent ( EPC  Patent Application No. 84112534  ). The

- 3 -

0150066

aqueous solution of monomeric human IFN-γ as obtained by said method contains the reducing sulfur compound and protein-denaturing agent and, among these low-molecular compounds, the protein-denaturing agent, in particular, should be removed since it cannot be used in pharmaceutical preparations. However, the aqueous solution obtained after treatment for removal of said agent proved not very suited for use as a raw material for the manufacture of pharmaceutical preparations. The reason is that the human IFN-γ contained therein has a low solubility and easily precipitates out during concentration or other treatment.

The present inventors conducted further intensive study, cleared up the cause of the decrease in solubility of human IFN-γ, and has now established a method for producing highly concentrated aqueous human IFN-γ solutions and obtained highly solubilized human IFN-γ in the solution.

Thus, the present invention provides a method for producing a high-concentrated human γ-interferon aqueous solution, which comprises removing a protein denaturing agent from a dilute aqueous solution of human γ-interferon containing the protein denaturing agent, aging the resulting solution in situ, and concentrating the same as well as an aqeuous human γ-interferon solution containing noncovalent dimer of γ-interferon.

As the above aqueous human IFN-γ solution which contains a protein-denaturing agent, preferably used is an aqueous human IFN-γ solution obtained by subjecting

crude human IFN-γ to gel filtration under the coexistence of a reducing sulfur compound and a protein-denaturing agent.

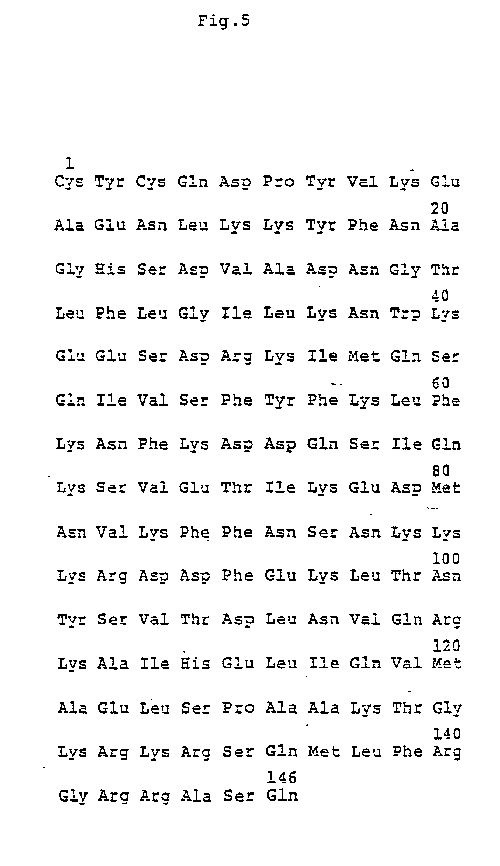

Said crude human IFN-γ to be purified by the method mentioned above may be any human IFN-γ-containing material. For instance, there may be used a crude product obtained by concentration naturally occurring human IFN-γ, i.e. natural IFN-γ(nIFN-γ) and a human IFN-γ-containing material produced by cultivating a human IFN-γ-producing microorganism obtained in turn by the gene mainpulation technology [cf. European Patent Publication No. 0 089 676; Nucleic Acids Research, 10, 2,487-2,501 (1982); Nature, 295, 503-508 (1982); Nucleic Acids Research, 10, 3605-3615 (1982)], i.e. recombinant IFN-γ (rIFN-γ). More concretely, the above-mentioned rIFN-γ includes polypeptide consisting of 146 amino acids, for example, of the sequence shown in Figure 5 and various fragments of the polypeptide, such as N terminal portion-deficient species, i.e. lacking not more than four amino acids of the N terminal part of the polypeptide and C terminal portion-deficient species which are cleaved at a site not earlier than the 131st amino acid residue of the polypeptide or the N terminal portion-deficient species. Furthermore, the rIFN-γ includes a polypeptide the cystein residues of the polypeptide being replaced by serine or theonine.

The polypeptide consisting 146 amino acid shown in Figure 5 and $Cys^1$-$Tyr^2$-$Cys^3$-deficient species of the polypeptide [des($Cys^1$-$Tyr^2$-$Cys^3$)IFN-γ] are preferred among others.

The diluted aqueous solution can be obtained, for example, by diluting a solution of IFN-γ in the monomeric state with a buffer to a concentration of 30-200 μg/ml using care not to cause aggregation or precipitation. Any other methods which will not cause aggregation or precipitation may also be used. Since dilution with a buffer of pH 5.0-8.0 alone generally results in immediate formation of white precipitates, a buffer containing a protein-denaturing agent in a concentration of 0.5 - 4 M is preferably used for dilution, whereby a diluted solution having no tendency to aggregation or precipitation can be obtained. The above buffer may further contain a reducing sulfur compound when IFN-γ contains a cystain residue.

The protein-denaturing agent includes, among others, guanidine salts (hydrochloride, sulfate), urea and thiocyanates (sodium salt, potassium salt).

The protein-denaturing agent can be removed by subjecting the above diluted aqueous solution to gel filtration or ultrafiltration, preferably by gel filtration.

The gel filtration is suitably carried out by the conventional column method.

The solution to be subjected to gel filtration is preferably the above diluted aqueous human IFN-γ solution.

Nevertheless, it may contain a precipitate in small amount if the gel filtration will not be disturbed thereby. Partial precipitation may occur during gel filtration, but the precipitate can be removed, for example, by filtration using a 0.22 μ membrane.

The gel to be used in said gel filtration can be selected optionally from among commercially available gels. Preferred are granular gels such as dextran, polyacrylamide and agarose. Particularly preferred are Sephadex G-25, Trisacryl GF-05 and the like which are suited for removing low-molecular compounds.

The gel is used generally in an amount of 2-100 times (v/v), preferably 5-20 times (v/v), the amount of the sample to be treated.

The developing solvent to be used is a buffer generally having a pH of 5.0-8.0, preferably a pH around neutrality, and preferably contains a reducing sulfur compound in a concentration of 1-100 mM, more preferably 5-20 mM when IFN-γ contains cystein residue.

More concretely, a gel column equilibrated in advance with the developing solvent is loaded with the above diluted aqueous solution. Elution is performed with the developing solvent. The rate of elution depends on the impurity content in the sample and the kind and amount of the gel, and is generally within an SV (space velocity) range of 0.1-10, preferably 0.5-3. The eluate is fractionated in the conventional manner.

Human IFN-γ-containing fractions can be easily detected by the conventional method, for example by examining an elution curve constructed based on O.D. 280 nm absorption data, among others.

The aging, in a liquid state, of the diluted aqueous solution after removal of the protein-denaturing agent can be effected by allowing said diluted solution to stand at an adequate temperature for an appropriate period of time.

The temperature and time factors in conducting the aging may be selected in any adequate manner. While about 4°C, is preferred from the viewpoint possibility of contamination with microorganisms and 35°C-40°C from the view point of the aging efficiency, an adequate temperature may possibly be selected within the range of about 0°C-40°C. As for the aging time, clouding may be encountered if gel filtration is immediately followed by concentration, but such clouding will be very slight already after an hour of standing. Generally 0.5-7 days, preferrably 24-72 hours of standing is sufficient for the concentration which follows.

As necessary, such an operation as sterile filtration may be performed prior to or during aging.

The concentration can advantageously be performed by ultrafiltration, for instance. The ultrafiltration membrane preferably has an MWCO of about 10,000 and the ultrafiltration can be conducted in the conventional manner.

The aqueous human IFN-γ solution produced by the above method contains human IFN-γ in a concentration of 0.2-1.5 mg/ml. It is thus a high concentration aqueous solution containing solubilized human IFN-γ as compared with the solution obtained by the above-mentioned prior method which has a concentration of about 0.05 mg/ml. Therefore, it is suited for use as an intermediate in large scale production of human IFN-γ.

The solubilization of human IFN-γ which has been achieved by the present invention is presumably due to the following.

Thus, it is presumable that the raw material human IFN-γ obtained by gel filtration of crude human IFN-γ under the coexistence of a reducing sulfur compound and a protein-denaturing agent is monomeric both covalently and noncovalently but turns noncovalently polymeric upon removal of the protein-denaturing agent. The aging in the state of diluted aqueous solution presumably causes conversion of the once-formed human IFN-γ polymer to a stable noncovalent dimer, whereby solubilization is achieved and easy concentration to a high concentration becomes possible.

The high concentration aqueous human IFN-γ solution produced by the method according to the invention has a higher concentration as compared with the aqueous human IFN-γ solution obtained by the above-mentioned prior method and therefore is used more advantageously as a raw solution for the manufacture of storable frozen products or lyophilized pharmaceutical preparations for, e.g. injection.

The human IFN-γ produced according to the invention has low toxicity and can be used for the same purposes and in the same modes of use as the conventional IFN-γ products. Since it has antiviral, antitumor, antiproliferative, immunopotentiating and other activities, it can be administered in the same manner as the known IFN-γ products.

The protein-denaturing agent-containing aqueous human IFN-γ solution, which is the starting material for practicing the invention, can be produced, for example, by subjecting crude human IFN-γ to gel filtration under the coexistence of a reducing sulfur compound and a protein-denaturing agent.

Said crude human IFN-γ may be any human IFN-γ-containing material. For instance, there may be used a product obtained by concentrating and isolating naturally occurring human IFN-γ or human IFN-γ produced by cultivating a human IFN-γ-prodcuing microorganism obtained by the gene manipulation technology [cf. European Patent Publication No. 0 089,676; Nucleic Acids Research, 10, 2,487-2,501 (1982); Nature, 295, 503-508 (1982)]. From the practical standpoint, a cell extract, an ammonium sulfate fractionation product, and antibody column eluate or an ion exchange eluate each obtained by cultivation of the above human IFN-γ-producing microorganism is used as the crude IFN-γ. However, since a larger amount of foreign proteins uneconomically requires a larger amount of reducing sulfur compound, the use of an antibody column eluate or ion exchange column eluate is generally preferred. In such crude material, human IFN-γ may occur in polymeric forms.

Said reducing sulfur compound includes organic sulfur-containing compounds, such as cysteine, N-acetylcysteine, N-acetylhomocysteine, glutathione (reduced form), thioethanolamine, monothioglycerol, dithiothreitol, thioalkanes of 1-7 carbon atoms and Rongalit, and inorganic sulfur-containing compounds, such as metabi-sulfites (sodium salt, potassium salt).

Said protein-denaturing agent includes those mentioned hereinabove.

The gel to be used in said gel filtration can be selected optionally from among commercially available ones, among others. Preferred are granular gels such as dextran, polyacrylamide and agarose. In treating human IFN-γ produced by a gene manipulation technique, for instance, a gel capable of fractionating within the molecular weight range of about 1,000-80,000 is preferably selected and used in view of the molecular weight of recombinant IFN-γ of 17,143 and the efficiency of separation from foreign proteins. Typical examples of

0150066

such gel are Sephadex G-50 and G-75, Sephacryl S-200 and Biogel P-10, P-30 and P-60.

The gel is used generally in an amount of 5-100 times (w/w), preferably 10-30 times (w/w), the amount of the sample to be treated.

The gel filtration is suitably carried out by the conventional column method.

Thus, crude human IFN-γ is dissolved in a buffer, for instance, and a gel column equilibrated in advance with a developing solvent is loaded with the aqueous solution. Elution is conducted with the developing solvent. The rate of elution depends on the impurity content in the sample and the kind and amount of the gel but, generally, an SV (space velocity) of 0.1-10, preferably 0.5-3, is employed.

The reducing sulfur compound and protein-denaturing agent may be made coexistent with human IFN-γ in any step of the above gel filtration process. It is preferred, however, to add the reducing sulfur compound and protein-denaturing agent to the aqueous human IFN-γ solution to be used for loading the gel therewith and to the developing solvent.

In cases where a protein-denaturing agent is used already in a step of pretreatment, such as extraction or antibody column treatment, then the aqueous solution to be treated on the gel column can be used as it is without further addition of such agent.

The above aqueous solution for loading and the developing solvent preferably have a pH of 5.0-8.0, more preferably a pH around neutrality, and preferably contain the reducing sulfur compound in a concentration of 1-100 mM, more preferably 5-20 mM and the protein-denaturing agent in a concentration of 0.1-7 M, more preferably 1-2 M.

The IFN-γ activity as described herein in terms of antiviral activity in international units (IU) was determined by subjecting an international standard IFN-γ sample with an established potency in units and the sample in question to the Sindbis virus-induced cytopathic effect prevention test in the human amnion-derived FL cell line, followed by calculation of the activity based on comparison between the potency data obtained. The protein amount in solution was determined by calculation on the assumption that E: 280 nm = 1.0 is equivalent to 1 mg.

The following examples and reference examples illustrate the invention in more detail. It is to be noted, however, that they are by no means limitative of the invention.

The antibody column Ab (Mo γ2-11.1) described in the reference examples was prepared by the method disclosed in European Patent Publication No. 0 103 898.

Brief Description of the Drawings

Fig. 1, 2 and 3 illustrate the results of gel filtration performed in Example 7 (2), (3) and Example 10 (2), respectively. The IFN-γ sample to be mesured is represented by ●, while standard proteins are represented by o (A: chymotrypsinogen A; B: ovalbumin; C: bovine serum albumin; D: cytochrome C).

Fig. 4 illustrates the construction scheme of the expression plasmid pLC2 disclosed in Reference Example 5.

Fig. 5 illustrates the amino acid sequence of IFN-γ consisting of 146 amino acid.

Example 1

To a 26-ml portion (protein content: 9.85 mg) of the IFN-γ (monomeric)-containing eluate (530 ml) obtained in Reference Example 2 (II), there was added 174 ml of a 25 mM acetate buffer diluent (pH 6.0) containing 10 mM cysteine hydrochloride, 150 mM sodium chloride, 0.5 M guanidine hydrochloride and 0.01% Tween 20, followed by stirring. Thus was prepared a low concentration solution with a protein content of 0.05 mg/ml. A Sephadex G-25 column (5 x 60 cm) equilibrated in advance with a 25 mM acetate buffer (pH 6.0) containing 10 mM cysteine hydrochloride, 150 mM sodium chloride and 0.01% Tween 20 was loaded with the above solution and elution was performed with the same buffer to give a guanidine hydrochloride-free, IFN-γ-containing eluate fraction (190 ml; protein content: 9.12 mg). The protein concentration of this solution was 0.048 mg/ml and the solution was clear and transparent. The protein recovery was 92.5%, and the specific IFN-γ activity was $3.7 \times 10^6$ IU/mg of protein.

Example 2

A 40-ml portion of the IFN-γ-containing eluate fraction (protein content: 1.92 mg) obtained in Example 1 was aged at 4°C for 24 hours and, then, concentrated to 4 ml by ultrafiltration using Diaflo PM-10, 43 mm ⌀ (Amicon's ultrafiltration membrane). The concentrate was clear and transparent and the protein concentration was 0.470 mg/ml. The protein recovery was 98% (1.88 mg). The specific IFN-γ activity was 6.8 x $10^6$ IU/mg of protein.

Example 3

A 40-ml portion (protein content: 1.92 mg) of the IFN-γ-containing eluate fraction obtained in Example 1 was aged at 4°C for 100 hours and thereafter concentrated to 4 ml in the same manner as in Example 2. The concentrate was clear and transparent and the protein concentration was 0.475 mg/ml. The protein recovery was 99% (1.9 mg). The specific IFN-γ activity was 7.3 x $10^6$ IU/mg of protein.

Example 4

Admixing of an IFN-γ-containing eluate fraction (510 ml; 0.388 mg/ml) obtained by the same method as used in Reference Example 2 (II) with 3,447 ml of the same diluent as used in Example 1 gave a solution having a protein concentration of 0.05 mg/ml. A Sephadex G-25 column (14 x 100 cm) equilibrated in advance with the same equilibrating buffer as used in Example 1 was loaded with the above solution, followed by elution with the same buffer. There was obtained 3,760 ml of IFN-γ eluate freed from guanidine hydrochloride. The protein concentration of this solution was 0.048 mg/ml and the specific IFN-γ activity was 3.5 x $10^6$ IU/mg of protein. The solution was aged at 4°C for 48 hours and then concentrated to 188 ml with Diaflo PM-10, 150 mm ⌀ to give a clear and transparent IFN-γ solution with a protein concentration of 0.96 mg/ml. Its specific IFN-γ activity was 6.7 x $10^6$

IU/mg of protein and convergence to the monomeric form was ascertained by SDS-PAGE.

Example 5

A low concentration solution having a protein concentration of 0.05 mg/ml was prepared by admixing the human IFN-γ (monomeric)-containing eluate fraction (450 ml) obtained in Reference Example 4 with 3,240 ml of a 25 mM acetate buffer diluent (pH 6.0) containing 10 mM glutathione (reduced form), 150 mM sodium chloride, 0.5M guanidine hydrochloride and 0.01% Tween 20. A Sephadex G-25 column (14 x 100 cm) euilibrated in advance with a 25 mM acetate buffer (pH 6.0) containing 10 mM glutathione (reduced form), 150 mM sodium chloride and 0.01% Tween 20 was loaded with the above solution. Gel filtration was conducted with the same buffer to give a human IFN-γ eluate fraction (3,180 ml; 155.8 mg) freed of guanidine hydrochloride. The protein concentration in this solution was 0.048 mg/ml. The protein recovery was 84.4% and the specific activity was $3.5 \times 10^6$ IU/mg of protein. This solution was aged at 4°C for 48 hours and, thereafter, concentrated to 159 ml by the same method as used in Example 2. The concentrate was clear and transparent and its protein concentration was 0.92 mg/ml. The protein recovery was 93.9% (146.3 mg). The specific human IFN-γ activity was $6.8 \times 10^6$ IU/mg of protein.

Example 6

A solution with a protein concentration of 0.05 mg/ml was prepared by adding 2,975 ml of a 25 mM acetate buffer diluent (pH 6.0) containing 150 mM sodium chloride, 0.5 M guanidine hydrochloride and 0.01% Tween 20 to 435 ml of a human IFN-γ (monomer) eluate fraction (0.392 mg protein/ml) obtained by the same method as used in Reference Example 2. A Sephadex G-25 column (14 x 100 cm) equilibrated in advance with a sufficiently nitrogen-purged 25 mM acetate buffer (pH 6.0) containing 150 mM sodium chloride and 0.01% Tween 20 was loaded with the above solution. Gel filtration was conducted with the same buffer to give a human IFN-γ eluate fraction (3,280 ml; 138 mg of protein) freed of the guanidine hydrochloride and glutathione (reduced form). The protein concentration in this solution was 0.042 mg/ml. The protein recovery was 81% and the specific human IFN-γ activity was $2.8 \times 10^6$ IU/mg of protein. This solution was aged at 4°C for 24 hours and, then, concentrated to 300 ml by the same method as in Example 2. The protein concentration of the concentrate was 0.239 mg/ml. The protein recovery was 52% (71.8 mg) and the specific human IFN-γ activity was $1.1 \times 10^6$ IU/mg of protein.

Example 7

The human IFN-γ obtained in Example 5 has the following characteristic properties.

(1) Molecular weight determination by sodium dodecylsulfate-polyacrylamide gel slab electrophoresis (SDS-PAGE)

Convergence to the monomeric form was ascertained at an apparent molecular weight of about 18,000.

Conditions: SDS concentration, 0.1%; acrylamide concentration 6% (gel for concentration), 12.5% (gel for separation); voltage, 60 V (in concentration), 180 V (in separation); concentration 60 minutes, separation 2.5-3.0 hours; color reagent, Coomassie Brilliant Blue.

(2)  Molecular weight determination by gel filtration

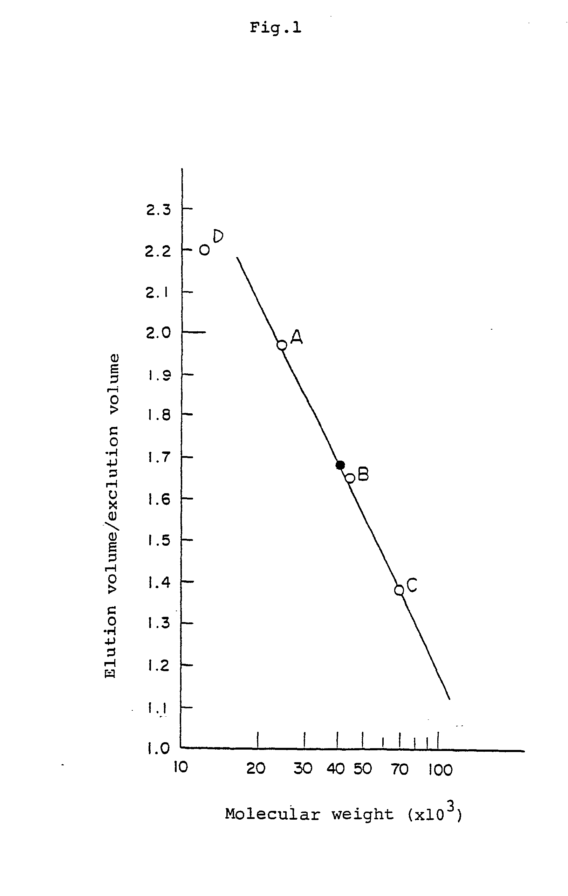

The apparent molecular weight was about 40,000 (Fig. 1).

Conditions:

(i)  Sample:  High concentration aqueous human IFN-γ solution according to the invention (protein concentration = 1.231 mg/ml; free of Tween 20) Standard proteins:  Bovine serum albumin (M.W. 68,000),  ovalbumin (M.W. 45,000), chymotrypsinogen A (M.W. 25,000) and cytochrome C (M.W. 12,500)

(ii) Gel filtration:  A Sephadex G-100 column (2.7 x 87 cm) equilibrated with a buffer (pH 6.0) containing 25 mM ammonium acetate, 150 mM NaCl and 10 mM glutathione (reduced form) was loaded with 2.46 mg/2 ml of sample and 5 mg/2 ml of each standard protein

**0150066**

and gel filtration was carried out at an elution rate of 21 ml/hour with fractionation by 4.5 ml portions.

(3) Molecular weight determination by gel filtration under the existence of SDS.

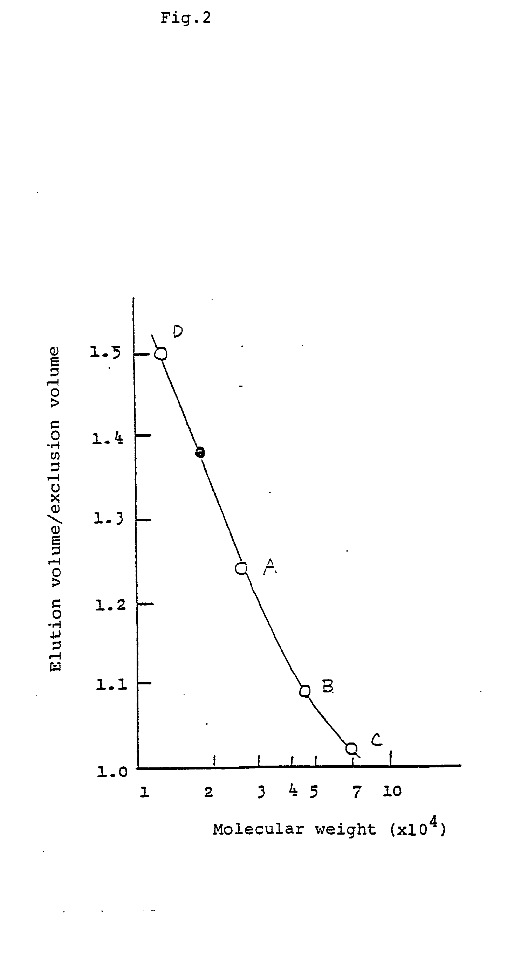

The apparant molecular weight was about 18,000 (Fig. 2).

Conditions:

(i) Sample: High concentration aqueous human IFN-γ solution according to the invention (protein concentration = 1.029 mg/ml; free of Tween 20) Standard proteins: Bovine serum albumin (M.W. 68,000), ovalbumin (M.W. 45,000), chymotrypsinogen A (M.W. 25,000) and cytochrome C (M.W. 12,500).

(ii) Gel filtration: A Sephadex G-100 column (2.7 x 87 cm) equilibrated with a buffer (pH 6.0) containing 25 mM ammonium acetate, 150 mM NaCl, 10 mM glutathione (reduced form) and 0.1% SDS was loaded with 2.06 mg/2 ml of sample and 5 mg/2 ml of each standard protein and gel filtration was carried out at an elution rate of 21 ml/hour with fractionation by 4.5 ml portions.

In SDS-PAGE and gel filtration under the existence of SDS, the human IFN-γ obtained in Example 5 was detected as a monomer (apparent molecular weight about 18,000) and, in gel filtration, it was detected as a dimer (apparent molecular weight about 40,000). It was thus revealed that said IFN-γ is a dimer composed of subunits each having a molecular weight of about 18,000 and that the bonding is noncovalent. It means that the human γ-interferon is covalently monomeric and noncovalently dimeric.

Example 8

200 ml solution having a protein concentration of 0.059 mg/ml was prepared by admixing 36 ml of human IFN-γ (monomeric)-containing eluate fraction (0.322 mg/ml) obtained by the method according to Reference Example 4 with 164 ml of a 25 mM acetate buffer diluent (pH 6.0) containing 10 mM glutathione (reduced form), 150 mM sodium chloride, 0.5M guanidine hydrochloride. A Sephadex G-25 column (5 x 60 cm) equilibrated in advance with a 25 mM acetate buffer (pH 6.0) containing 10 mM glutathione (reduced form) and 150 mM sodium chloride was loaded with the above solution. Gel filtration was conducted with the same buffer to give a human IFN-γ eluate fraction (218 ml) freed of guanidine hydrochloride. The protein concentration in this solution was 0.045 mg/ml. The eluate was aged at 37°C for 3 days after sterile filtration using Acro 50 A (0.2 μm; Gelman). The resulting solution was concentrated to 10 ml by ultrafiltration using Diaflo PM-10 (Amicon's ultrafiltration membrane). The concentrate was clear and transparent and the protein concentration was 0.905 mg/ml. The concentrate showed convergence to the monomeric form in SDS-PAGE. The specific human IFN-γ activity was 37 x $10^6$ IU/mg of protein.

Example 9

To 2.2 ml of eluate (protein content: 0.331 mg/ml) containing des($Cys^1$-$tyr^2$-$cys^3$) IFN-γ obtained in Reference Example 5 (iii), there was added 8 times amount of a 25 mM acetate buffer diluent (pH 6.0) containing 150 mM sodium chloride and 2 M guanidine hydrochloride followed by stirring. Thus was prepared a low concentration solution. A Sephadex G-25 column (2.6 x 15 cm) equilibrated in advance with a 25 mM acetate buffer (pH 6.0) containing 150 mM sodium chloride was loaded with the above solution and elution was performed with the same buffer to give a guanidine hydrochloride-free des($Cys^1$-$tyr^2$-$cys^3$) IFN-γ-containing eluate fraction (30 ml). The protein concentration of this solution was 0.022 mg/ml and the solution was clear and transparent.

This eluate fraction was agend at 4°C for 24 hours and, then, concentrated to 0.68 ml by ultrafiltration using Diaflo YM-10, 25 mm φ (Amicon's ultrafiltration, membrane, and filtered using a filter (0.2 μm) to obtain 0.68 ml of clear and transparent solution. The protein concentration was 0.670 mg/ml. The protein recovery was 63%.

Example 10

The des($Cys^1$-$tyr^2$-$cys^3$) IFN-γ obtained in Example 9 has the following characteristic properties.

(1)    Molecular weight determination by SDS-PAGE

Convergence to the monomeric form was ascertained at an apparent molecular weight of about 17,000.

Conditions: SDS concentration, 0.1%; acrylamide concentration 4% (gel for concentration), 12.5% (gel for separation); voltage, 60 V (in concentration), 180 V (in separation); concentration 60 minutes, separation 3.0 hours; color reagent, Coomassie Brilliant Blue.

(2) Molecular weight determination by gel filtration

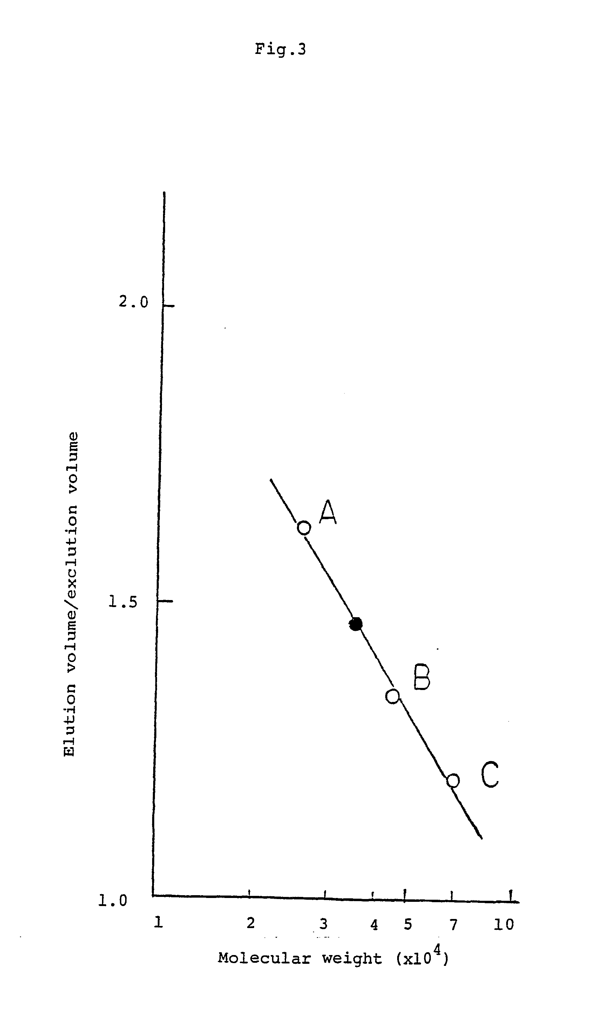

The apparent molecular weight was about 35,000. (Fig. 3).

Conditions:

(i) Sample: High concentration aqueous des(Cys$^1$-Tyr$^2$-Cys$^3$)IFN-γ solution obtained in Example 9 (protein concentration = 0.670 mg/ml) Standard proteins: Bovine serum albumin (M.W. 68,000), ovalbumin (M.W. 45,000) and chymotrypsinogen A (M.W. 25,000)

(ii) Gel filtration: A Sephadex G-100 column (1.0 x 55 cm) equilibrated with a buffer (pH 6.0) containing 25 mM ammonium acetate and 150 mM NaCl was loaded with 0.27 mg/0.4 ml of sample and 1 mg/0.4 ml of each standard protein and gel filtration was carried out at a flow rate of 2 ml/hour with fractionation by 0.5 ml portions.

On SDS-PAGE, des(Cys[1]-Tyr[2]-Cys[3]) IFN-γ obtained in Example 9 was detected as a monomer (apparent molecular weight about 17,000) and, on gel filtration, it was detected as a dimer (apparent molecular weight about 35,000). It was thus revealed that said IFN-γ is a dimer composed of subunits each having a molecular weight of about 17,000 and that the bonding is noncovalent.

Reference Example 1

The strain RRI (pRK 248 cIts, pRC 231/IFI-900) carrying the human IFN-γ expression gene as described in Example 8 of European Patent Publication No. 0 089 676 was cultivated in M9-glucose medium at 30°C until the cell concentration reached 3-4 x $10^8$ cells/ml. Glucose and casamino acids were then added in concentration of 1.0% and 0.5%, respectively. After an hour of induction at 42°C, the culture was centrifuged and the cells thus collected were frozen and stored.

Reference Example 2

(I) To 1,000 g of the forzen cells obtained in Reference Example 1 was added 3,000 ml of 100 mM Tris hydrochloride buffer (pH 7.0) containing 7 M guanidine hydrochloride and 2 mM phenylmethylsulfonyl fluoride. The mixture was stirred at 4°C for 1 hour and centrifuged (17,000 rpm/30 minutes). The clear and transparent supernatant thus obtained was diluted 70-fold with a buffer comprising 137 mM sodium chloride, 2.7 mM potassium chloride, 8.1 mM disodium hydrogen phosphate and 1.5 mM potassium dihydrogen phosphate (hereinafter abbreviated as PBS). The resulting precipitate was removed using a Sharples centrifuge (10,000 rpm). The supernatant obtained (220 liters) was concentrated to 15 liters using a Pericon membrane filter (Millipore Corp.; molecular weight cut-off 10,000). The concentrate was allowed to stand at 4°C overnight and the resulting precipitate was removed by further centrifugation on a Sharples centrifuge. A preliminarily packed antibody column [Ab (Mo γ2-11.1); 5 x 30 cm] was loaded with the above supernatant at a flow rate of 1,000 ml/hour. Thereafter, washing solutions, namely 2,500 ml of PBS, 5,000 ml of 10 mM phosphate buffer (pH 7.0) containing 1 M sodium chloride and 0.1% Tween 20, 2,500 ml of PBS and 2,500 ml of 20 mM phosphate buffer (pH 7.0) containing 0.5 M guanidine hydrochloride, were passed through the column in that order, followed by elution with a 20 mM phosphate buffer (pH 7.0) containing 2 M guanidine hydrochloride. There was collected 500 ml of eluate fraction having antiviral activity.

(II) Cysteine hydrochloride was added, in an amount to give a concentration of 10 mM, to the eluate fraction obtained in Reference Example 2 (I).

A Sephacryl S-200 (Pharmacia) column (9 x 100 cm) equilibrated in advance with a 25 mM acetate buffer (pH 6.0) containing 1 mM ethylenediaminetetraacetate, 150 mM sodium chloride, 10 mM cysteine hydrochloride and 2 M guanidine hydrochloride was loaded with the above human IFN-γ solution (500 ml), followed by elution with the same buffer. A monomer eluate fraction (530 ml) was thus collected. The thus-obtained fraction showed convergence to the monomeric form also in slab electrophoresis using sodium dodecylsulfate (SDS-PAGE). (Apparent molecular weight: about 18,000). This molecular sieve treatment gave 201 mg of human IFN-γ having a specific activity of $3.3 \times 10^6$ IU/mg.

Reference Example 3

A Sephadex G-25 column (5 x 60 cm) equilibrated in advance with an equilibrating 25 mM acetate buffer (pH 6.0) containing 10 mM cysteine hydrochloride, 150 mM sodium chloride and 0.01% Tween 20 was loaded with a 200-ml (75.8 mg) portion of the human IFN-γ (monomer) eluate fraction (530 ml; 0.379 mg/ml) obtained in Reference Example 2 (II), followed by elution with the same buffer, whereby 180 ml of human IFN-γ eluate fraction freed of guanidine hydrochloride was obtained. With this eluate, clouding occurred immediately after elution, giving a precipitate. Therefore, the eluate was subjected to centrifugation (10,000 rpm/30 minutes). Measurement of the protein content in the supernatant gave a value of 0.05 mg/ml. The protein recovery was 11.9% (9 mg). The specific human IFN-γ activity was $3.3 \times 10^6$ IU/mg of protein.

Reference Example 4

Glutathione (reduced form) was added, in an amount to give a concentration of 10 mM, to an eluate fraction (420 ml) obtained by the method of Reference Example 2 (I).

A Sephacryl S-200 (Pharmacia) column (9 x 100 cm) equilibrated in advance with a 25 mM acetate buffer (pH 6.0) containing 1 mM ethylenediaminetetraacetate, 150 mM sodium chloride, 10 mM glutathione (reduced form) and 2 M guanidine hydrochloride was loaded with the above aqueous human IFN-γ solution (420 ml), followed by elution with the same buffer, whereby 450 ml of monomer eluate fraction was collected. The above procedure gave human IFN-γ (0.410 mg/ml) having a specific activity of $3.4 \times 10^6$ IU/mg of protein.

Reference Example 5   Production of des(Cys$^1$-Tyr$^2$-Cys$^3$) IFN-γ

(i)   Transformant production

The IFN-γ expression plasmid pRC23/IFI-900 [cf.
Example 7 of the specification for a patent application
under EPC as laid open under No. 0089676] was digested
with the restriction enzymes NdeI and NcoI, and a 710 bp
NdeI-NcoI DNA fragment (A) containing the IFN-γ gene region
was isolated.  Separately, the plasmid pRC23 was digested
with the restriction enzymes BglII and EcoRI, and a 265 bp
DNA fragment (B) containing the λP$_L$ promoter was isolated.
The fragments (A) and (B) and the chemically synthesized,
protein synthesis start codon-containing oligonucleotide AATTCATGCAGGATCCA

GTACGTCCTAGGTAT

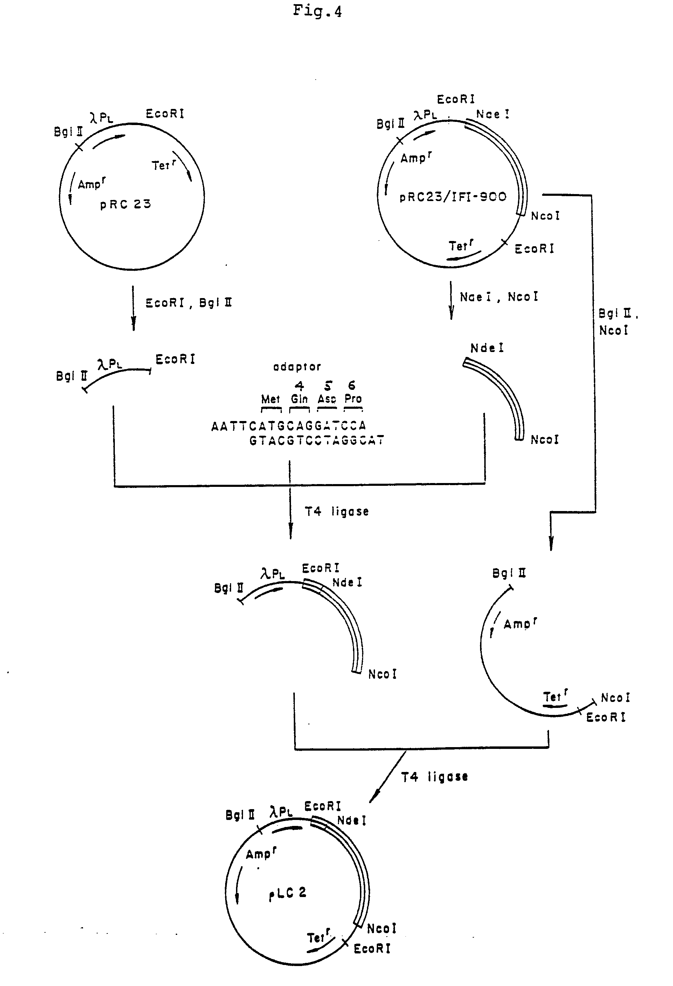

were joined together using T4 DNA ligase, with the NdeI
and EcoRI cohesive ends as the sites of joining.  The DNA
fragment thus obtained was joined to the plasmid pRC23/IFI-
900 after treatment with NcoI and BglII, to thereby construct an expression plasmid, pLC2, coding for the Cys$^1$-
Tyr$^2$-Cys$^3$-deficient IFN-γ polypeptide (Fig. 4).  This plasmid
pLC2 was used for transforming Escherichia coli RRI(pRK248
cIts) by the method of Cohen et al.[P.N.A.S. (USA), 69,
2110 (1972)] to give a transformant, Escherichia coli
(=E. coli) PRI(pLC2,pRK248 cIts).

(ii)   Transformant cultivation

The strain E. coli RRI(pLC2,pRK248 cIts) carrying
the plasmid constructed in (i) above was shake-cultured at 35°C in 50 ml of a liquid medium containing 1% Bacto-tryptone, 0.5% yeast extract, 0.5% sodium chloride and 7 μg/ml tetracycline. The culture broth was transferred to 2.5 liters of M9 medium containing 0.5% casamino acids, 0.5% glucose and 7 μg/ml tetracycline, and grown at 35°C for 4 hours and then at 42°C for 3 hours. Cells were harvested by centrifugation and stored at -80°C.

(iii) Purification

In 22 ml of 0.1 M Tris-hydrochloride buffer (pH 7.0) containing 7 M guanidine hydrochloride and 2 mM phenylmethylsulfonyl fluoride, there were suspended 7.1 g of frozen cells obtained in the same manner as mentioned above in (ii). The suspension was stirred at 4°C for 1 hour and then centrifuged at 10,000 x g for 30 minutes to give 24 ml of a supernatant. This supernatant was diluted by adding 300 ml of PBS and the dilution was applied to an antibody column (Moγ2-11.1, column capacity 15 ml) at a flow rate of 1 ml/minute. Thereafter, the column was washed with 60 ml of 20 mM sodium phosphate buffer (pH 7.0) containing 0.5 M guanidine hydrochloride and then eluted with 45 ml of 20 mM sodium phosphate buffer (pH 7.0) containing 2 M guanidine hydrochloride, to give 25 ml of an antivirally active fraction. This fraction (25 ml) was applied to a Sephacryl S-200 (Pharmacia) column (2.6 x 94 cm; column capacity 500 ml) equilibrated in advance with 25 mM ammonium acetate buffer (pH 6.0) containing 1 mM ethylenediaminetetraacetic acid, 0.15 M sodium chloride, 10 mM cysteine and 2 M guanidine hydrochloride, and eluted with the same buffer to give 40 ml of an antivirally active fraction.

The thus-obtained Cys-Tyr-Cys-deficient IFN-γ poly-peptide des($\overset{1}{C}$ys-$\overset{2}{T}$yr-$\overset{3}{C}$ys) IFN-γ weighed 7.0 mg and had a specific activity of $2.7 \times 10^6$ IU/mg.

Reference Example 6

A Sephadex G-25 column equilibrated in advance with 25 mM ammonium acetate buffer (pH 6.0) containing 150 mM sodium chloride was loaded with 1.2 ml (0.621 mg/ml) portion of des(Cys$^1$-Tyr$^2$-Cys$^3$) IFN-γ eluate fraction obtained in Reference Example 5 (iii), followed by elution with the same buffer, whereby the INF-γ eluate fraction freed of guanidine hydrochloride was obtained. With this eluate, clouding occurred immediately after elution, giving a precipitate. Therefore, the eluate was subjected to filtration using a filter (0.2 μm) to obtain 2.4 ml of clear and tranparent solution containing 0.150 mg/ml of protein. The protein recovery was 48%.

What is claimed is:

1.  A method for producing a highly concentrated human γ-interferon aqueous solution, which comprises removing a protein-denaturing agent from a diluted aqueous solution of human γ-interferon containing the protein-denaturing agent, aging the resulting solution in situ and concentrating the same.

2.  The method according to claim 1, wherein the human γ-interferon in the highly concentrated human γ-interferon aqueous solution is noncovalent dimer.

3.  The method according to claim 1, wherein the human γ-interferon is recombinant human γ-interferon.

4.  The method according to claim 1, wherein the human γ-interferon is a polypeptide consisting of 146 amino acids shown in Figure 5.

5.  The method according to claim 1, wherein the human γ-interferon is des(Cys$^1$-Tyr$^2$-Cys$^3$) γ-interferon.

6.  The method according to claim 1, wherein the diluted aqueous solution of human γ-interferon containing the protein-denaturing agent is a diluted eluate obtained by subjecting crude human γ-interferon to gel filtration under the coexistence of a reducing sulfur compound and a protein-denaturing agent with a buffer containing the protein-denaturing agent.

7.  The method according to claim 6, wherein the human γ-interferon in the diluted aqueous solution is in a concentration of 30-200 µg/ml.

8.  The method according to claim 1, wherein the protein-denaturing agent is guanidine salt.

9.  The method according to claim 8, wherein the guanidine salt is guanidine hydrochloride.

10.  The method according to claim 1, wherein the diluted aqueous solution of human γ-interferon contains the protein-denaturing agent in a concentration of 0.1 to 7 M.

11. The method according to claim 1, wherein the protein-denaturing agent is removed by subjecting the diluted aqueous human γ-interferon solution containing the protein-denaturing agent to gel filtration.

12. The method according to claim 11, wherein the gel is a member selected from a group consisting of dextran gel polyacrylamide gel and agarose gel.

13. The method according to claim 11, wherein the gel is dextran gel.

14. The method according to claim 11, wherein the gel filtration is conducted by loading the diluted aqueous human γ-interferon solution on a column packed with the gel and then eluting the γ-interferon by a buffer solution.

15. The method according to claim 14, the buffer solution is of pH 5.0 to 8.0.

16. The method according to claim 14, wherein the buffer solution contains reducing sulfur compound.

17. The method according to claim 14, wherein the elution is conducted at a space velocity of 0.1 to 10.

18. The method according to claim 1, wherein the solution is agend in situ at a temperature of 0° to 40°C for 0.5 to 7 days.

19. The method according to claim 18, wherein the solution is aged in situ at a temperature of 35° to 40°C for 24 to 72 hours.

20. The method according to claim 1, wherein the concentration of the aged solution is conducted by ultrafiltration.

21 An aqueous human γ-interferon solution which contains noncovalent dimer of human γ-interferon.

22. The aqueous solution according to claim 21, wherein the human γ-interferon is a polypeptide consisting of 146 amino acids shown in Figure 5.

23. The aqueous solution according to claim 22, wherein the human γ-interferon shows an apparent molecular weight of about 40,000 in gel filtration under the non-existence
of sodium dodecylsulfate, and of about 18,000 in sodium
dedecylsulfate-polyacrylamide gel slab electrophoresis
and in gel filtration under the existence of sodium
dodecylsulfate.

24. The aqueous solution according to claim 21, wherein
the human γ-interferon is des (C$\overset{1}{y}$s-T$\overset{2}{y}$r-C$\overset{3}{y}$s) γ-interferon.

25. The aqueous solution according to claim 24, wherein the
human γ-interferon shows an apparent molecular weight of
about 35,000 in gel filtration under the non-existence
of sodium dodecylsulfate, and of about 17,000 in sodium
dodecylsulfate-polyacrylamide gel slab electrophoresis.

Fig.1

0150066

Elution volume/exclution volume

Molecular weight (x10³)

Fig.2

Elution volume/exclusion volume (y-axis): 1.0, 1.1, 1.2, 1.3, 1.4, 1.5

Molecular weight (x10$^4$) (x-axis): 1, 2, 3, 4, 5, 7, 10

Points labeled D, a, A, B, C

Fig.3

Fig.4

Fig.5

1
Cys Tyr Cys Gln Asp Pro Tyr Val Lys Glu

20
Ala Glu Asn Leu Lys Lys Tyr Phe Asn Ala

Gly His Ser Asp Val Ala Asp Asn Gly Thr

40
Leu Phe Leu Gly Ile Leu Lys Asn Trp Lys

Glu Glu Ser Asp Arg Lys Ile Met Gln Ser

60
Gln Ile Val Ser Phe Tyr Phe Lys Leu Phe

Lys Asn Phe Lys Asp Asp Gln Ser Ile Gln

80
Lys Ser Val Glu Thr Ile Lys Glu Asp Met

Asn Val Lys Phe Phe Asn Ser Asn Lys Lys

100
Lys Arg Asp Asp Phe Glu Lys Leu Thr Asn

Tyr Ser Val Thr Asp Leu Asn Val Gln Arg

120
Lys Ala Ile His Glu Leu Ile Gln Val Met

Ala Glu Leu Ser Pro Ala Ala Lys Thr Gly

140
Lys Arg Lys Arg Ser Gln Met Leu Phe Arg

146
Gly Arg Arg Ala Ser Gln